# EUROPÄISCHE PATENTSCHRIFT

(11) **EP 3 335 281 B1**
(45) Veröffentlichungstag und Bekanntmachung des Hinweises auf die Patenterteilung: **29.05.2019**
(21) Anmeldenummer: 16729493.3
(22) Anmeldetag: 14.06.2016
(51) Int. Cl.: H01R 12/88, H01R 12/73, H01R 13/24, H01R 13/42, H01R 13/50, A61B 5/055

(54) **BOARD-TO-BOARD-VERBINDER ZUM SIGNALÜBERTRAGENDEN VERBINDEN VON ZWEI LEITERPLATTEN**
BOARD-TO-BOARD CONNECTOR FOR SIGNAL-TRANSMITTING CONNECTION OF TWO CIRCUIT BOARDS
CONNECTEUR CARTE À CARTE ETABLISSANT UNE CONNEXION ELECTRIQUE ENTRE DEUX CIRCUITS IMPRIMÉS

(30) Priorität: 13.08.2015 DE 202015005722 U
(43) Veröffentlichungstag der Anmeldung: 20.06.2018
(73) Patentinhaber: Rosenberger Hochfrequenztechnik GmbH & Co. KG, 83413 Fridolfing (DE)
(72) Erfinder: HOFMEISTER, Stefan, 83417 Kirchanschöring (DE); RÄTHLEIN, Martin, 83416 Saaldorf-Surheim (DE); MICHELMANN, Folke, 84529 Tittmoning (DE); SCHICHL, Markus, 5201 Seekirchen (AT)
(74) Vertreter: Zeitler Volpert Kandlbinder Patent- und Rechtsanwälte Partnerschaft mbB
(86) Internationale Anmeldenummer: PCT/EP2016/000994
(87) Internationale Veröffentlichungsnummer: WO 2017/025162

(56) Entgegenhaltungen:
- EP-A1- 1 536 524
- JP-A- 2001 068 184
- US-A- 5 161 981
- US-A1- 2008 171 451

## Beschreibung

Die Erfindung betrifft einen Board-to-Board-Verbinder zum signalübertragenden Verbinden von zwei Leiterplatten miteinander, insbesondere eines Magnetresonanztomographen.

Board-zu-Board-Verbinder (englisch board-to-board connectors oder BTB connectors) werden verwendet, um zwei Leiterplatten (englisch printed circuit board, PCB) so miteinander zu verbinden, dass elektronische Komponenten auf den beiden Leiterplatten durch Austausch von elektrischen Signalen miteinander kommunizieren können.

Zum signalübertragenden Verbinden von zwei Leiterplatten miteinander ist es bekannt, ein Verbindungsteil, wie ein Kontaktpin, mit einem Gegenstück, wie einer Buchse, durch Stecken zu verbinden, wobei z.B. die Buchse auf einer der Leiterplatten aufgelötet wird. Jedoch ist das Auflöten eines derartigen Gegenstücks aufwendig. Beim Steckvorgang kann der Kontaktpin Schaden nehmen, sodass er irreparabel verformt wird und ein Bilden einer Steckverbindung nicht mehr möglich ist. Dies hat einen technischen Ausfall der betroffenen Leiterplatte zur Folge. Zudem werden Leiterplatten ohne zusätzliche mechanische Halterung miteinander verbunden. Erschütterungen und Vibrationen können daher zu einer Unterbrechung des elektrischen Kontaktes bzw. der Kontakte führen.

Die EP 1 536 524 A1 offenbart einen Board-to-Board-Verbinder mit Federelementen zum elektrischen Verbinden von zwei Leiterplatten. Die Leiterplatten können in ein Gehäuse des Board-to-Bord Verbinders eingeschoben werden.

Die US 2008/0171451 A1 beschreibt einen Board-to-Board-Verbinder zum Verbinden von in einer Ebene angeordneten Leiterplatten.

Die JP 2001 068184 A offenbart ebenfalls einen Board-to-Board-Verbinder zum Verbinden von in einer Ebene angeordneten Leiterplatten.

Die US 5 161 981 A beschreibt einen Pressverbinder bzw. Hochdruckverbinder, bei welchem Leiterspuren tragende flexible Kabel von entgegengesetzten Seiten an einen nachgiebigen Isolatorkern gepresst werden, wobei der nachgiebige Isolatorkern von einem dünnen Film mit Leiterspuren umgeben ist.

Der Erfindung liegt daher die Aufgabe zugrunde, einen Weg aufzuzeigen, wie das signalübertragende Verbinden von zwei Leiterplatten vereinfacht und zugleich weniger fehleranfällig ausgebildet werden kann.

Diese Aufgabe wird erfindungsgemäß durch einen Board-to-Board-Verbinder der o.g. Art mit den in Anspruch 1 genannten Merkmalen gelöst. Vorteilhafte Ausgestaltungen der Erfindung sind in den weiteren Ansprüchen beschrieben.

Der erfindungsgemäße Board-to-Board-Verbinder zum signalübertragenden Verbinden von einer ersten Leiterplatte mit einer zweiten Leiterplatte weist einen Grundkörper mit einer ersten Aufnahme für einen Kontaktabschnitt der ersten Leiterplatte und mit einer zweiten Aufnahme für einen Kontaktabschnitt der zweiten Leiterplatte auf, wobei zwischen der ersten Aufnahme und der zweiten Aufnahme ein Federelementträger mit einer Mehrzahl von Federelementen vorgesehen ist, wobei jedes der Federelemente je einen Kontakt der jeweiligen Kontaktabschnitte unter Federandruckkraft elektrisch leitend kontaktiert.

Dies hat den Vorteil, dass zum einen keine aufwändige Lötverbindung gebildet werden muss, was den Fertigungsaufwand reduziert, und dass zum anderen durch den Federanpressdruck auf empfindliche Stifte zum Bilden einer Steckverbindung verzichtet werden kann. So wird der Fertigungsaufwand reduziert und zugleich die Fertigung weniger fehleranfällig ausgebildet. Ferner wird ein kraftfreies und reibungsloses Stecken zum Verbinden der beiden Leiterplatten möglich, was die Lebensdauer der Oberflächen der Leiterplatten erhöht. Des Weiteren wird durch die mechanische Fixierung ein Zerstören der Kontaktflächen der Kontakte der beiden Leiterplatten während des Steckvorgangs verhindert, wobei ferner aufgrund der mechanischen Fixierung die Baugruppe, bestehend aus den beiden Leiterplatten und dem Board-to-Board-Verbinder, weniger anfällig gegenüber Erschütterungen oder Vibrationen ist. Schließlich ist mit dem Board-to-Board-Verbinder ein werkzeugloses Verbinden von zwei Leiterplatten miteinander möglich.

Gemäß einer Ausführungsform ist die erste Aufnahme eine erste Einführrichtung für den Kontaktabschnitt der ersten Leiterplatte vorgebend ausgebildet und die zweite Aufnahme ist eine zweite Einführrichtung für den Kontaktabschnitt der zweiten Leiterplatte vorgebend ausgebildet, wobei die erste Einführrichtung und die zweite Einführrichtung voneinander verschieden sind. Z.B. können die erste Einführrichtung und die zweite Einführrichtung nahezu entgegengesetzt sein. So wird ein besonders einfach zu handhabender Board-to-Board-Verbinder bereitgestellt.

Gemäß einer weiteren Ausführungsform sind die erste Aufnahme und die zweite Aufnahme jeweils als Einschiebefach ausgebildet. So kann der Board-to-board-Verbinder besonders einfach und zugleich besonders einfach zu montieren ausgebildet sein, was die Fertigung nochmals vereinfacht.

Die erste Aufnahme ist dem Federelementträger zugeordnet. Weiter ist die erste Aufnahme durch eine an dem Federelementträger angeformte Führung gebildet. So vereinfacht sich die

Fertigung der Bauteile, da die erste Aufnahme und die zweite Aufnahme jeweils unterschiedlichen Bauteilen zugeordnet sind.

Die zweite Aufnahme ist einem schwenkbaren an dem Grundkörper angelenkten Deckel zugeordnet, der zwischen einer offenen und einer geschlossenen Position verlagerbar ist. Weiter ist die zweite Aufnahme durch eine an dem Deckel angeformte Führung gebildet. Der angelenkte Deckel kann z.B. mittels eines Filmscharniers an dem Grundkörper befestigt sein. Er ist somit unverlierbar mit dem Grundkörper verbunden.

Gemäß einer weiteren Ausführungsform weist der Board-to-Board-Verbinder zumindest einen Zapfen auf, der derart ausgebildet ist, das ein Deckel des Grundkörpers nur bei korrekt in die erste Aufnahme und/oder zweiten Aufnahme eingesetzten Kontaktabschnitten von seiner offenen in seine geschlossen Position verlagerbar ist. So werden Fehler bei der Montage zuverlässig vermieden, bei denen aufgrund einer Fehlplatzierung der ersten und/oder zweiten Leiterplatte in der ersten bzw. zweiten Aufnahme eine elektrische Kontaktierung der Kontakte des Kontaktabschnitts mit den jeweiligen Federelementen nicht gegeben ist.

Gemäß einer weiteren Ausführungsform weist der Grundkörper zumindest einen Zapfen auf, der an dem Deckel angeformt ist. So kann ein besonders einfach zu fertigender Grundkörper verwendet werden.

Gemäß einer weiteren Ausführungsform ist der ersten Aufnahme und/oder der zweiten Aufnahme ein Gegenfederelement zugeordnet. Das jeweilige Gegenfederelement ist gegenüberliegend dem Federelementträger angeordnet, wobei jeweils ein Spalt zwischen dem Federelementträger und dem jeweiligen Gegenfederelement die erste bzw. zweite Aufnahme definiert. Durch das Gegenfederelement wird der Anpressdruck und damit die Verbindungsqualität der elektrischen Kontakte erhöht.

Gemäß einer weiteren Ausführungsform ist der Board-to-Board-Verbinder aus unmagnetischen Materialien gefertigt. Dabei werden unter unmagnetischen Materialien Materialien mit einer Permeabilitätszahl µᵣ von ungefähr 1 verstanden. Es kann sich z.B. um paramagnetische oder diamagnetische Materialien handeln. Dies erlaubt es, den Board-to-Board-Verbinder einer Umgebung mit starken magnetischen Feldern einzusetzen, z.B. in einem Magnetresonanztomographen.

Ferner gehört zur Erfindung eine Baugruppe, aufweisend eine erste Leiterplatte und eine zweite Leiterplatte, die durch einen derartigen Board-to-Board-Verbinder signalübertragend miteinander verbunden sind. Die Baugruppe kann ferner Rahmen aufweisen, die als Schutzrahmen der Leiterplatten Kanten und/oder Ecken der Leiterplatten vor Schäden schützen.

Die Erfindung wird im Folgenden anhand der Zeichnung näher erläutert. Diese zeigt in
- Fig. 1: eine Explosionsdarstellung eines Ausführungsbeispiel eines erfindungsgemäßen Board-to-Board-Verbinders,
- Fig. 2: in schematischer Darstellung ein Sicherungselement des in Fig. 1 gezeigten Board-to-Board-Verbinders,
- Fig. 3: in schematischer Darstellung einen Grundkörper des in Fig. 1 gezeigten Board-to-Board-Verbinders ,
- Fig. 4: in schematischer Darstellung einen ersten Schritt der Montage des in Fig. 1 gezeigten Board-to-Board-Verbinders,
- Fig. 5: in schematischer Darstellung einen zweiten Schritt der Montage des in Fig. 1 gezeigten Board-to-Board-Verbinders,
- Fig. 6: in schematischer Darstellung einen dritten Schritt der Montage des in Fig. 1 gezeigten Board-to-Board-Verbinders, und
- Fig. 7: in schematischer Darstellung den in Fig. 1 gezeigten Board-to-Board-Verbinder mit geschlossenem Deckel.

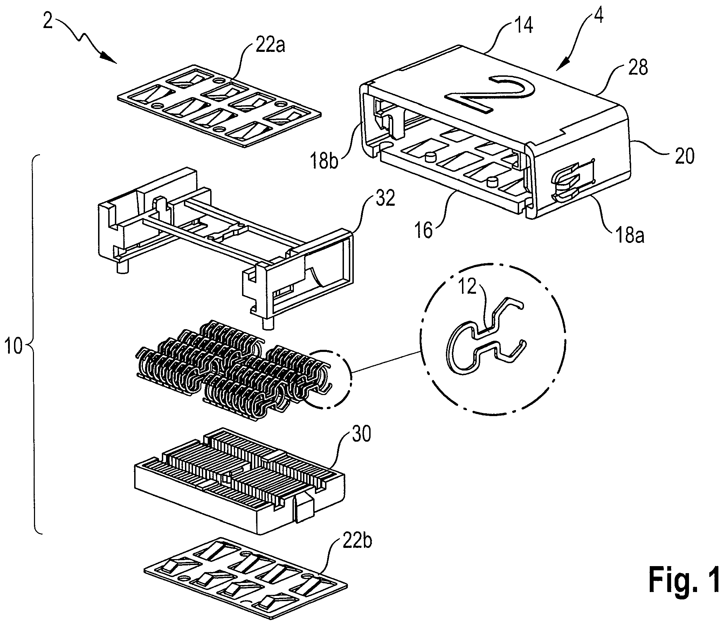

Es wird zunächst auf Fig. 1 Bezug genommen.

Der in Fig. 1 gezeigte Board-to-Board-Verbinder 2 ist zum elektrisch leitenden Verschalten von einer ersten Leiterplatte mit einer zweiten Leiterplatte ausgebildet, um Kontakte eines Kontaktabschnitt der ersten Leiterplatte mit Kontakten eines Kontaktabschnitts der zweiten Leiterplatte elektrisch leitend zu verbinden, so dass die erste Leiterplatte und die zweite Leiterplatte zusammen mit dem Board-to-Board-Verbinder 2 eine Baugruppe bilden.

Die Baugruppe kann eine Komponente eines Magnetresonanztomographen (MR bzw. MRT) sein, wobei eine der beiden Leiterplatten z.B. zur Steuerung einer justierbaren Patientenliege und die andere Leiterplatte zur Ansteuerung eines Röhrenkörpers ausgebildet sind. Eine oder beide Leiterplatten können einen Rahmen aufweisen, der als Schutzrahmen dient, um insbesondere Ecken oder Kanten der Leiterplatten vor Beschädigungen zu schützen.

Der Board-to-Board-Verbinder 2 weist im vorliegenden Ausführungsbeispiel einen Grundkörper 4, einen Federelementträger 10 und zwei Gegenfederelemente 22a, 22b auf.

Der Grundkörper 4 ist im vorliegenden Ausführungsbeispiel ein einstückig ausgebildetes Kunststoffspritzgussteil mit einer im Wesentlichen quaderförmigen Grundform. Der Grundkörper 4 weist einen Deckel 14, einen Boden 16, zwei Seitenwände 18a, 18b und eine Rückwand 20 auf.

Der Deckel 14 ist über ein als Filmscharnier ausgebildetes Schwenkgelenk 28 an dem Grundkörper 4 zwischen einer offenen Position und einer geschlossenen Position verlagerbar unverlierbar befestigt.

Zur Anordnung im Inneren des Grundkörpers 4 ist ein Federelementträger 10 vorgesehen. Der Federelementträger 10 weist im vorliegenden Ausführungsbeispiel einen Basiskörper 30, eine Mehrzahl von Federelementen 12 und ein Sicherungselement 32 auf. Bei dem Basiskörper 30 und dem Sicherungselement 32 handelt es sich im vorliegenden Ausführungsbeispiel um einstückig ausgebildete Kunststoffspritzteile, während es sich bei den Federelementen 12 um Goldbauteile oder um Bauteile aus einer Gold enthaltenden Legierung handelt.

Ferner ist je ein Gegenfederelement 22a, 22b zum Befestigen an einer Innenseite des Deckels 14 und an einer Innenseite des Bodens 16 vorgesehen. Die Gegenfederelement 22a, 22b sind im vorliegenden Ausführungsbeispiel jeweils als Niederhalterbleche ausgebildet. Bei den Gegenfederelementen 22a, 22b handelt es sich im vorliegenden Ausführungsbeispiel je um ein gestanztes und gebogenes bzw. abgekantetes Blech, das eine Mehrzahl von Federzungen aufweist.

Im vorliegenden Ausführungsbeispiel ist der Board-to-Board-Verbinder 2 mit seinen Bestandteilen Grundkörper 4, Federelementträger 10 (mit dem Basiskörper 30 und dem Sicherungselement 32 sowie der Mehrzahl an Federelementen 12) und den Gegenfederelementen 22a, 22b jeweils aus einem unmagnetischen, z.B. paramagnetischen und/oder diamagnetischen Material mit einer Permeabilitätszahl µᵣ von ungefähr 1 gefertigt.

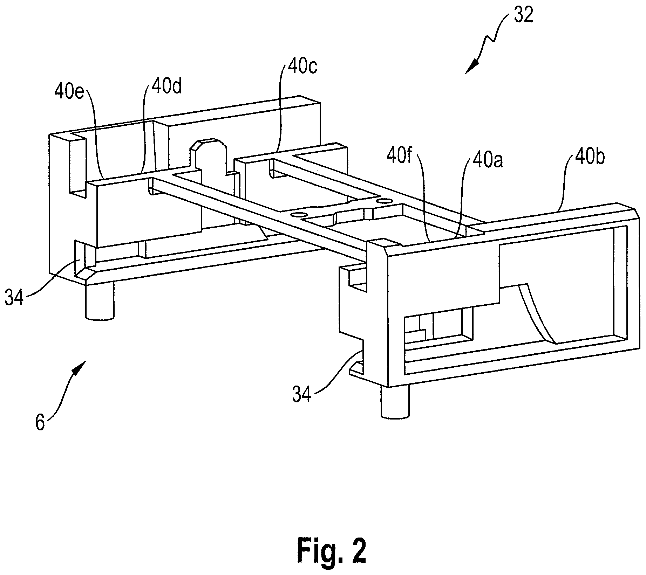

Es wird nun zusätzlich auf Fig. 2 Bezug genommen.

In Fig. 2 ist das Sicherungselement 32 des Federelementträgers 10 gezeigt.

Das Sicherungselement 32 weist eine angeformte erste Führung 34 auf, die aus zwei sich gegenüberliegenden Führungsschienen besteht. Die erste Führung 34 definiert eine erste A ufnahme 6 für einen Kontaktabschnitt der ersten Leiterpla tte. Unt er einem Kontaktabschnitt einer Leiterplatte wird dabei der Abschnitt der Leiterplatte verstanden, in dem Kontakte angeordnet sind, die elektrisch leitend mit den Federelementen 12 verbunden werden sollen, während auf anderen Abschnitten der Leiterplatte verschiedene elektronische Bauteile angeordnet sind.

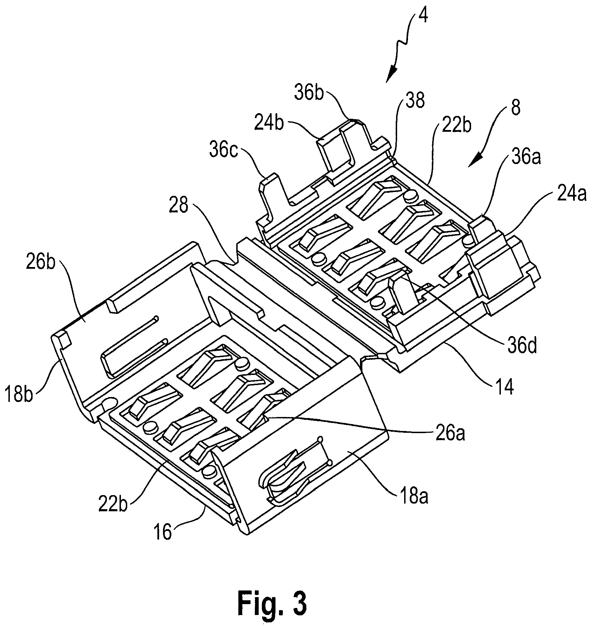

Es wird nun zusätzlich auf Fig. 3 Bezug genommen.

In Fig. 3 ist der Grundkörper 4 mit dem Deckel 14 in seiner geöffneten Position gezeigt. Dabei sind an der Innenseite des Deckels 14 und an der Innenseite des Bodens 16 die Gegenfederelemente 22a, 22b bereits befestigt.

Zur Fixierung des Deckels 14 in seiner geschlossenen Position sind im vorliegenden Ausführungsbeispiel an dem Deckel 14 Rasthaken 24a, 24b angeformt, die in der geschlossenen Position in Eingriff mit korrespondierenden Vertiefungen 26a, 26b des Grundkörpers 4 gebracht werden können, um den Deckel 14 so in der geschlossenen Position zu fixieren.

Ferner weist der Deckel 14 eine angeformte zweite Führung 38 auf, die aus zwei sich gegenüberliegenden Führungsschienen besteht. Die zweite Führung 38 definiert eine zweite Aufnahme 8 für einen Kontaktabschnitt der zweiten Leiterplatte.

Des Weiteren weist der Deckel 14 angeformete Zapfen 36a, 36b, 36c, 36d auf, die als Kodierzapfen ausgebildet sind, während das Sicherungselement 32 (siehe Fig. 2) die Zapfen 36a, 36b, 36c, 36d und die Rasthaken 24a, 24b des Deckels 14 aufnehmende Zapfenaufnahmen 40a, 40b, 40c, 40d, 40e, 40f aufweist, in die die Zapfen 36a, 36b, 36c, 36d und Rasthaken 24a, 24b eingreifen, wenn der Federelementträger 10 mit dem Sicherungselement 32 in den Grundkörper 4 eingesetzt ist und der Deckel 14 sich in seiner geschlossenen Position befindet.

Der Grundkörper 4 mit dem Deckel 14, dem Boden 16, den zwei Seitenwänden 18a, 18b, der Rückwand 20, der ersten Führung 34 und den Zapfen 36a, 36b, 36c, 36d ist dabei im vorliegenden Ausführungsbeispiel ein einstückig ausgebildetes Kunststoffspritzgussteil.

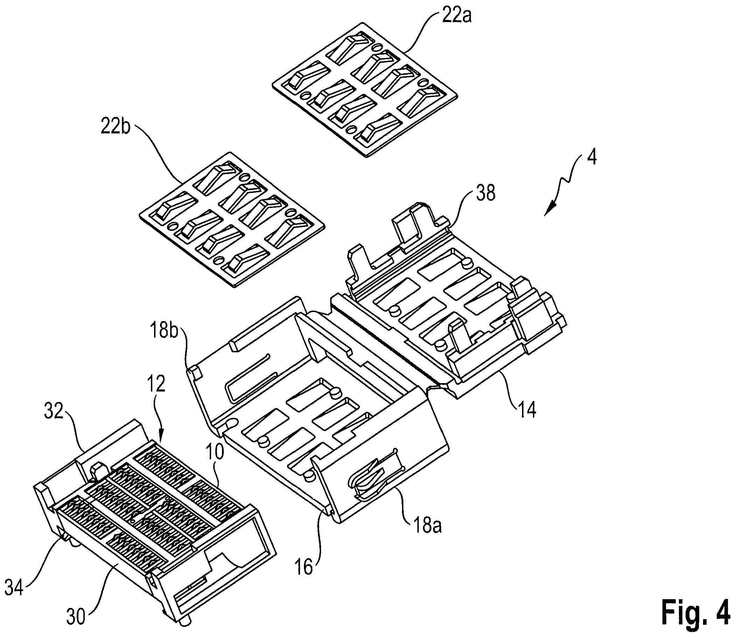

Es wird nun unter zusätzliche Bezugnahme auf die Fig. 4 bis 7 die Montage des Board-to-Board-Verbinders 2 erläutert.

Der Federelementträger 10 wurde bereits vormontiert, indem in Aufnahmen für die Federelemente 12 die Federelemente 12 eingesetzt und dann der Federelementträger 10 mit dem Sicherungselement 32 verbunden wurde. Das Sicherungselement 32 ist derart ausgebildet, dass es Einführöffnungen zum Einsetzen der Federelemente 12 in die Aufnahmen verschließt, so dass die Federelemente 12 in den Aufnahmen gesichert sind. Dabei sind die Federelemente 12 in den Aufnahmen schwimmend gelagert.

In einem ersten Schritt (siehe Fig. 4) werden bei geöffneten Deckel 14 die beiden Gegenfederelemente 22a, 22b an der Innenseite des Deckels 14 und an der Innenseite des Bodens 16 befestigt, im vorliegenden Ausführungsbeispiel durch Heißverstemmen.

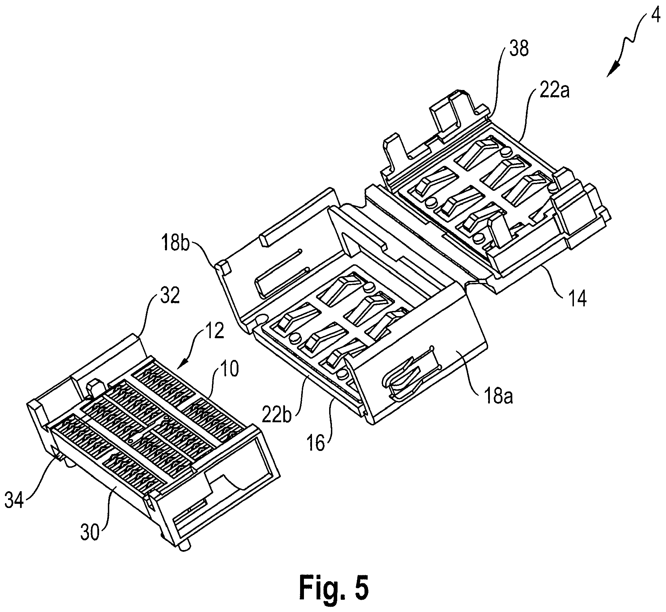

In einem zweiten Schritt (siehe Fig. 5) wird ebenfalls bei geöffneten Deckel 14 der vormontierte Federelementträger 10 mit dem Grundkörper 4 verbunden, in dem der Federelementträger 10 zwischen den Seitenwänden 18a, 18b eingesetzt und im vorliegenden Ausführungsbeispiel durch Heißverstemmen fixiert wird.

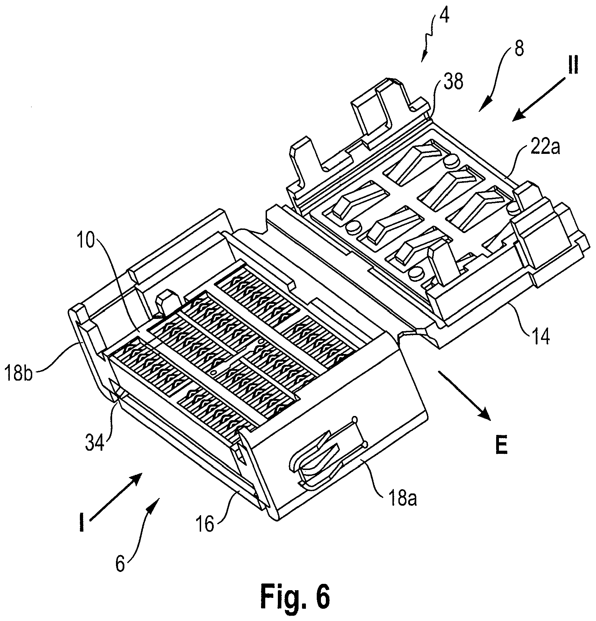

In einem dritten Schritt (siehe Fig. 6) werden ein Kontaktabschnitt der ersten Leiterplatte durch eine Einführbewegung entlang der ersten Einführrichtung I in die erste Aufnahme 6 und ein Kontaktabschnitt der zweiten Leiterplatte durch eine Einführbewegung entlang der zweiten Einführrichtung II in die zweite Aufnahme 8 eingeführt. Dabei wird im vorliegenden Ausführungsbeispiel zuerst in einem ersten Teilschritt der Kontaktabschnitt der ersten Leiterplatte durch eine Einführbewegung entlang der ersten Einführrichtung I in die erste Aufnahme 6 und dann in einem zweiten Teilschritt der Kontaktabschnitt der zweiten Leiterplatte durch eine Einführbewegung entlang der zweiten Einführrichtung II in die zweite Aufnahme 8 eingeführt.

Im vorliegenden Ausführungsbeispiel ist die erste Einführrichtung I und die zweite Einführrichtung II jeweils ein seitliches Einschieben. Die erste Aufnahme 6 und die zweite Aufnahme 8 sind als Einschiebefächer ausgebildet, die beabstandet voneinander ausgebildet sind.

Somit sind die erste Einführeinrichtung I und die zweite Einführrichtung II voneinander verschieden. Im vorliegenden Ausführungsbeispiel sind die erste Einführeinrichtung I und die zweite Einführrichtung II entgegensetzt zueinander. Ferner weisen im vorliegenden Ausführungsbeispiel die erste Einführeinrichtung I und die zweite Einführrichtung II einen Winkel von im Wesentlichen 90° zu einer Erstreckungsrichtung E der Schwenkachse des Schwenkgelenks 28 auf. Unter "im Wesentlichen" wird dabei innerhalb von fertigungsbedingten Toleranzen liegend verstanden.

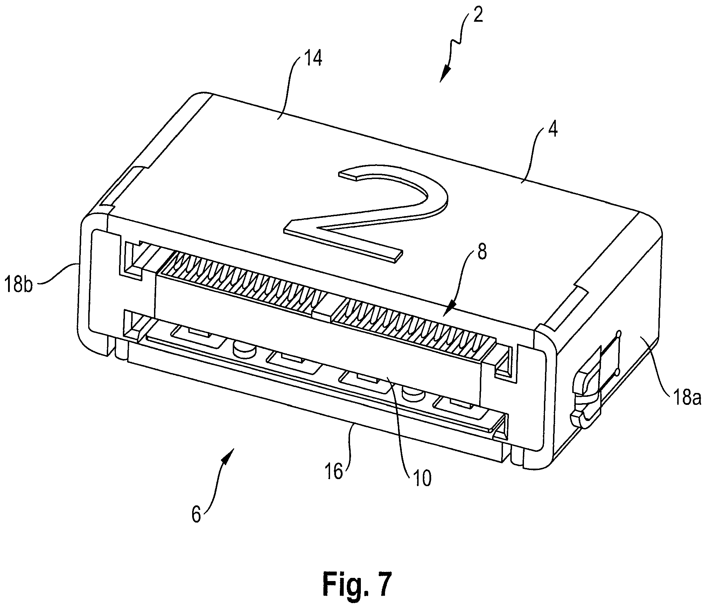

In einem vierten Schritt (siehe Fig. 7) wird der Deckel 14 mit dem Federelementträger 10 von seiner offenen Position in seine geschlossene Position gebracht. Hierbei tauchen die Zapfen 36a, 36b, 36c, 36d und die Rasthaken 24a, 24b des Deckels 14 in die korrespondierenden Zapfenaufnahmen 40a, 40b, 40c, 40d, 40e, 40f des Federelementträgers 10 ein.

Ferner zeigt Fig. 7, dass zwischen dem Federelementträger 10 und dem Gegenfederelementen 22a, 22b je ein Spalt gebildet ist, der die erste Aufnahme 6 zur Aufnahme des Kontaktabschnitt der ersten Leiterplatte und die zweite Aufnahme 8 zur Aufnahme des Kontaktabschnitt der zweiten Leiterplatte definiert.

Die Zapfen 36a, 36b, 36c, 36d und die Rasthaken 24a, 24b des Deckels 14 sind dabei so ausgebildet, dass ein Schließen des Deckels 14 und Verrasten der Rasthaken 24a, 24bnur dann möglich ist, wenn die erste Leiterplatte korrekt in die erste Aufnahme 6 und die zweite Leiterplatte korrekt in die zweite Aufnahme 8 eingeschoben wurde, da dies im vorliegenden Ausführungsbeispiel ein Auslenken der Zapfen 36a, 36b, 36c, 36d und der Rasthaken 24a, 24b bewirkt.

Nun werden Kontaktabschnitte der ersten Leiterplatte von dem Gegenfederelement 22a an die Federelemente 12 des Federelementträger 10 gepresst, so dass ein sicherer, elektrisch leitender Kontakt der Kontakte des Kontaktabschnitts der ersten Leiterplatte mit den jeweiligen Federelementen 12 gewährleistet ist.

Zugleich werden Kontaktabschnitte der zweiten Leiterplatte von dem Gegenfederelement 22b an die Federelemente 12 des Federelementträgers 10 gepresst, so dass ein sicherer, elektrisch leitender Kontakt der Kontakte des Kontaktabschnitts der zweiten Leiterplatte mit den jeweiligen Federelementen 12 gewährleistet ist. So werden die jeweiligen Kontakte der Kontaktabschnitte durch die entsprechenden Federelemente 12 elektrisch leitend verbunden.

Abweichend von dem anhand der Fig. 4 bis 7 erläuterten Ausführungsbeispiel für die Montage des Board-to-Board-Verbinders 2 kann die Reihenfolge der Schritte auch variieren, z.B. kann die Reihenfolge des ersten Schritts und des zweiten Schritts vertauscht sein.

Allerdings kann im vorliegenden Ausführungsbeispiel die erste Leiterplatte in die erste Aufnahme 6 nur bei geöffneten Deckel 14 eingeschoben werden. Andernfalls versperren die Zapfen 36a, 36b, 36c, 36d den Einführweg in die ersten Aufnahme 6. Somit ist bei dem vorliegenden Ausführungsbeispiel die erste Leiterplatte zuerst, d.h. vor der zweiten Leiterplatte, zu stecken bzw. einzuschieben. Dies schließt eine verkehrte Steckreihenfolge, d.h. zuerst ein Einstecken der zweiten Leiterplatte in die zweite Aufnahme und dann ein Einstecken der ersten Leiterplatte in die erste Aufnahme, aus.

Somit können mit dem Board-to-Board-Verbinder 2 zwei Leiterplatten ohne eine Lötverbindung herzustellen signalübertragend miteinander verbunden werden und es können keine empfindlichen Stifte Schäden nehmen.

## Patentansprüche

1. Board-to-Board-Verbinder (2) zum signalübertragenden Verbinden von einer ersten Leiterplatte mit einer zweiten Leiterplatte, insbesondere eines Magnetresonanztomographen, wobei der Board-to-Board-Verbinder (2) einen Grundkörper (4) mit einer ersten Aufnahme (6) für einen Kontaktabschnitt der ersten Leiterplatte und mit einer zweiten Aufnahme (8) für einen Kontaktabschnitt der zweiten Leiterplatte aufweist, wobei zwischen der ersten Aufnahme (6) und der zweiten Aufnahme (8) ein Federelementträger (10) mit einer Mehrzahl von Federelementen (12) vorgesehen ist, wobei jedes der Federelemente (12) je einen Kontakt der jeweiligen Kontaktabschnitte unter Federandruckkraft elektrisch leitend kontaktiert, **dadurch gekennzeichnet, dass** die zweite Aufnahme (8) einem an dem Grundkörper (4) schwenkbaren angelenkten Deckel (14) zugeordnet ist, der zwischen einer offenen und einer geschlossenen Position verlagerbar ist, wobei die zweite Aufnahme durch eine an dem Deckel angeformte Führung gebildet ist, und dass die erste Aufnahme (6) dem Federelementträger (10) zugeordnet ist, wobei die erste Aufnahme durch eine an dem Federelementträger angeformte Führung gebildet ist.

2. Board-to-Board-Verbinder (2) nach Anspruch 1, **dadurch gekennzeichnet, dass** die erste Aufnahme (6) eine erste Einführrichtung (I) für den Kontaktabschnitt der ersten Leiterplatte vorgebend ausgebildet ist, die zweite Aufnahme (8) eine zweite Einführrichtung (II) für den Kontaktabschnitt der zweiten Leiterplatte vorgebend ausgebildet ist, wobei die erste Einführrichtung (I) und die zweite Einführrichtung (II) voneinander verschieden sind.

3. Board-to-Board-Verbinder (2) nach Anspruch 1 oder 2, **dadurch gekennzeichnet, dass** die erste Aufnahme (6) und die zweite Aufnahme (8) jeweils als Einschiebefach ausgebildet sind.

4. Board-to-Board-Verbinder (2) nach einem der vorstehenden Ansprüche, **dadurch gekennzeichnet, dass** der Board-to-Board-Verbinder (2) zumindest einen Zapfen (36a, 36b, 36c, 36d) aufweist, der derart ausgebildet ist, dass ein Deckel (14) des Grundkörpers (4) nur bei korrekt in die erste Aufnahme (6) und/oder zweiten Aufnahme (8) eingesetzten Kontaktabschnitten von seiner offenen in seine geschlossen Position verlagerbar ist.

5. Board-to-Board-Verbinder (2) nach Anspruch 4, **dadurch gekennzeichnet, dass** der zumindest eine Zapfen (36a, 36b, 36c, 36d) an dem Deckel (14) angeformt ist.

6. Board-to-Board-Verbinder (2) nach einem der vorstehenden Ansprüche, **dadurch gekennzeichnet, dass** der ersten Aufnahme (6) und/oder der zweiten Aufnahme (8) ein Gegenfederelement (22a, 22b) zugeordnet ist.

7. Board-to-Board-Verbinder (2) nach Anspruch 6, **dadurch gekennzeichnet, dass** zwischen dem Federelementträger (10) und den Gegenfederelementen (22a, 22b) Spalte gebildet sind, die die erste Aufnahme und die zweite Aufnahme definieren.

8. Board-to-Board-Verbinder (2) nach einem der vorstehenden Ansprüche, **dadurch gekennzeichnet, dass** der Board-to-Board-Verbinder (2) aus unmagnetischen Materialien gefertigt ist.

9. Board-to-Board-Verbinder (2) nach einem der vorstehenden Ansprüche, **dadurch gekennzeichnet, dass** der Deckel (14) angeformte Zapfen (36a, 36b, 36c, 36d) aufweist, die als Kodierzapfen ausgebildet sind, und die in der geschlossenen Position des Deckels (14) in Zapfenaufnahmen (40a, 40b, 40c, 40d, 40e, 40f) eingreifen.

10. Board-to-Board-Verbinder (2) nach einem der vorstehenden Ansprüche, **dadurch gekennzeichnet, dass** Zapfen (36a, 36b, 36c, 36d) des Deckels (14) zum Eintauchen in korrespondierende Zapfenaufnahmen (40a, 40b, 40c, 40d, 40e, 40f) des Federelementträgers (10) ausgebildet sind.

11. Baugruppe, insbesondere eines Magnetresonanztomographen, aufweisend eine erste Leiterplatte, eine zweite Leiterplatte und einen Board-to-Board-Verbinder (2) nach einem der vorstehenden Ansprüche, wobei die erste Leiterplatte und die zweite Leiterplatte durch den Board-to-Board-Verbinder (2) signalübertragend miteinander verbunden sind.

## Claims

1. Board-to-board connector (2) for the signal-transmitting connection of a first printed circuit board with a second printed circuit board, in particular of a magnetic resonance imaging scanner, wherein the board-to-board connector (2) has a main body (4) with a first socket (6) for a contact section of the first printed circuit board and a second socket (8) for a contact section of the second printed circuit board, wherein a spring element support (10) with a plurality of spring elements (12) is provided between the first socket (6) and the second socket (8), wherein each of the spring elements (12) makes electrically conductive contact with a contact of the respective contact sections under spring-loaded contact pressure, **characterised in that** the second socket (8) is assigned a pivotable cover (14) articulated to the main body (4) which can be moved between an open and a closed position, wherein the second socket is formed by a guide moulded onto the cover, and that the first socket (6) is assigned to the spring element support (10), wherein the first socket is formed by a guide moulded onto the spring element support..

2. Board-to-board connector (2) according to claim 1, **characterised in that** the first socket (6) is designed so as to predetermine a first insertion direction (I) for the contact section of the first printed circuit board, the second socket (8) being designed so as to predetermine a second insertion direction (II) for the contact section of the second printed circuit board, wherein the first insertion direction (I) and the second insertion direction (II) are different from one another.

3. Board-to-board connector (2) according to claim 1 or 2, **characterised in that** the first socket (6) and the second socket (8) are in each case designed in the form of a plug-in slot.

4. Board-to-board connector (2) according to one of the preceding claims, **characterised in that** the board-to-board connector (2) has at least one pin (36a, 36b, 36c, 36d) which is designed such that a cover (14) of the main body (4) can only be moved from its open position into its closed position if the contact sections are inserted correctly into the first socket (6) and/or second socket (8).

5. Board-to-board connector (2) according to claim 4, **characterised in that** the at least one pin (36a, 36b, 36c, 36d) is moulded onto the cover (14).

6. Board-to-board connector (2) according to one of the preceding claims, **characterised in that** a return spring element (22a, 22b) is assigned to the first socket (6) and/or the second socket (8).

7. Board-to-board connector (2) according to claim 6, **characterised in that** gaps are formed between the spring element support (10) and the return spring elements (22a, 22b) which define the first and the second socket.

8. Board-to-board connector (2) according to one of the preceding claims, **characterised in that** the board-to-board connector (2) is made of nonmagnetic materials.

9. Board-to-board connector (2) according to one of the preceding claims, **characterised in that** moulded onto the cover (14) are pins (36a, 36b, 36c, 36d) which are designed in the form of coding pins and which engage in pin sockets (40a, 40b, 40c, 40d, 40e, 40f) when the cover (14) is in its closed position.

10. Board-to-board connector (2) according to one of the preceding claims, **characterised in that** pins (36a, 36b, 36c, 36d) on the cover (14) are designed to engage in corresponding pin sockets (40a, 40b, 40c, 40d, 40e, 40f) on the spring element support (10).

11. Assembly, in particular of a magnetic resonance imaging scanner, comprising a first printed circuit board, a second printed circuit board and a board-to-board connector (2) according to one of the preceding claims, wherein the first printed circuit board and the second printed circuit board are connected with one another in a signal-transmitting manner by means of the board-to-board connector (2).

## Revendications

1. Connecteur carte à carte (2) pour la connexion avec transmission de signaux d'une première carte à circuits avec une seconde carte à circuits, en particulier d'un tomographe à résonance magnétique, le connecteur carte à carte (2) comprenant un corps de base (4) pourvu d'un premier logement (6) pour une portion de contact de la première carte à circuits et d'un second logement (8) pour une portion de contact de la seconde carte à circuits, un support d'élément ressort (10) étant prévu entre le premier logement (6) et le second logement (8) et présentant une pluralité d'éléments formant ressorts (12), chacun des éléments formant ressorts (12) mettant en contact électriquement conducteur un contact respectif des portions de contact sous une force d'application élastique, **caractérisé en ce que** le second logement (8) est associé à un couvercle (14) articulé avec faculté de basculement sur le corps de base (4) et mobile entre une position ouverte et une position fermée, le second logement étant formé par un guidage conformé sur le couvercle, et **en ce que** le premier logement (6) est associé au support d'élément ressort (10), le premier logement étant formé par un guidage conformé sur le support d'élément ressort.

2. Connecteur carte à carte (2) selon la revendication 1, **caractérisé en ce que** le premier logement (6) est réalisé de manière à imposer une première direction d'introduction (I) pour la portion de contact de la première carte à circuits, le second logement (8) est réalisé de manière à imposer une seconde direction d'introduction (II) pour la portion de contact de la seconde carte à circuits, la première direction d'introduction (I) et la seconde direction d'introduction (II) étant différentes l'une de l'autre.

3. Connecteur carte à carte (2) selon la revendication 1 ou 2, **caractérisé en ce que** le premier logement (6) et le second logement (8) sont réalisés chacun sous forme de casier d'introduction.

4. Connecteur carte à carte (2) selon l'une des revendications précédentes, **caractérisé en ce que** le connecteur carte à carte (2) comprend au moins un tenon (36a, 36b, 36c, 36d) qui est réalisé de telle sorte qu'un couvercle (14) du corps de base (4) est mobile depuis sa position ouverte jusque dans sa position fermée uniquement lorsque les portions de contact sont mises en place correctement dans le premier logement (6) et/ou dans le second logement (8).

5. Connecteur carte à carte (2) selon la revendication 4, **caractérisé en ce que** ledit au moins un tenon (36a, 36b, 36c, 36d) est conformé sur le couvercle (14).

6. Connecteur carte à carte (2) selon l'une des revendications précédentes, **caractérisé en ce qu'**un élément formant ressort antagoniste (22a, 22b) est associé au premier logement (6) et/ou au second logement (8).

7. Connecteur carte à carte (2) selon la revendication 6, **caractérisé en ce que** des fentes sont formées entre le support d'élément ressort (10) et les éléments formant ressorts antagonistes (22a, 22b), fentes qui définissent le premier logement et le second logement.

8. Connecteur carte à carte (2) selon l'une des revendications précédentes, **caractérisé en ce que** le connecteur carte à carte (2) est fabriqué en matériaux amagnétiques.

9. Connecteur carte à carte (2) selon l'une des revendications précédentes, **caractérisé en ce que** le couvercle (14) comprend des tenons (36a, 36b, 36c, 36d) conformés qui sont réalisés sous forme de tenons de codage et qui, dans la position fermée du couvercle (14), viennent s'engager dans des logements à tenon (40a, 40b, 40c, 40d, 40e, 40f).

10. Connecteur carte à carte (2) selon l'une des revendications précédentes, **caractérisé en ce que** des tenons (36a, 36b, 36c, 36d) du couvercle (14) sont réalisés pour plonger dans des logements à tenon correspondants (40a, 40b, 40c, 40d, 40e, 40f) du support d'élément ressort (10).

11. Groupe structurel, en particulier d'un tomographe à résonance magnétique, comportant une première carte à circuits, une seconde carte à circuits et un connecteur carte à carte (2) selon l'une des revendications précédentes, la première carte à circuits et la seconde carte à circuits étant connectées l'une à l'autre de manière à transmettre des signaux par le connecteur carte à carte (2).
